# EUROPEAN PATENT APPLICATION

(11) **EP 3 269 396 A2**
(43) Date of publication of application: **17.01.2018**
(21) Application number: 17176728.8
(22) Date of filing: 19.06.2017
(51) Int. Cl.: A61L 2/18, A47G 25/16, A47G 25/20, A61L 2/26, B08B 3/02, B08B 11/02, B08B 13/00, D06B 1/02, D06B 23/04, D06F 17/04

(54) **WASHING APPARATUS**

(30) Priority: 20.06.2016 IT UA20164499
(71) Applicant: Steelco S.p.A., 31039 Riese Pio X (IT)
(72) Inventor: Zardini, Fabio, 31033 Castelfranco Veneto (TV) (IT); Capovilla, Ivone, 31037 Loria (TV) (IT); Casonato, Ottorino, 31033 Castelfranco Veneto (TV) (IT)
(74) Representative: Petraz, Gilberto Luigi

(57) **Abstract**

Washing apparatus comprising at least a washing chamber (11) and at least a hydraulic circuit (33) to deliver at least a treatment fluid inside the washing chamber (11); the washing chamber (11) is configured to house at least a support device (12) comprising; at least a support unit (21), provided with a frame (22) in the form of a cage and able to support items (G1, G2) to be washed and at least a delivery unit (23) to deliver said treatment fluid configured to be hydraulically connected to this hydraulic circuit (33) and provided with tubular elements (24-27) configured to be positioned, at least partly, inside said frame (22), and therefore in the internal parts of the items (G1, G2) to be washed.

## Description

### FIELD OF THE INVENTION

Embodiments described here concern an apparatus for washing items, in particular items such as garments or suchlike, usable in the health field, in hospitals or suchlike, for example supported by frames or trolleyed supports.

### BACKGROUND OF THE INVENTION

Apparatuses for washing items, for example in hospitals, are known, comprising a washing chamber in which a trolleyed movement device is introduced on which the items to be washed are disposed.

The items are subjected, inside the washing chamber, to at least one of the following treatments, which constitute a so-called wash cycle: prewash in cold and/or hot water, hot water wash, advantageously with detergents, rinsing, chemical disinfection and/or thermodisinfection, with a possible final drying in air, hot air or other fluid.

The items, as we said, can also include garments used in the hospital, such as gowns used in radiology wards or suchlike, which require particular care in washing and/or thermodisinfection.

At present, the treatment or washing cycle does not guarantee that these garments are perfectly washed and/or disinfected, mainly due to the poor reliability of current washing apparatuses and the currently used washing and/or disinfection methods.

Known trolleyed movement devices, in which the clothes to be introduced into the washing chamber could be placed, have obvious limits to their processing efficiency, especially with regard to the internal parts of the garments, which the washing and/or thermodisinfection fluids used in the washing chambers have difficulty in reaching.

Consequently therefore, still today, garments washed by these known washing apparatuses do not have any guarantee or certification regarding the washing cycle to which they have been subjected in the washing chamber.

Clearly, even in the case of hand washing of such garments, it is not possible to obtain any certification on the type of washing carried out, with the further risk that the garments are not washed properly.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to obtain a washing apparatus which can overcome at least one of the disadvantages of the state of the art.

In particular, one purpose of the present invention is therefore to obtain a washing apparatus for items, for example, garments usable in hospitals, which ensures an efficient and reliable washing of said garments.

Another purpose of the present invention is to obtain an apparatus for washing items, for example, garments usable in hospitals, by means of which a guarantee certificate can be obtained concerning the washing cycle performed.

Another purpose of the present invention is to obtain a washing apparatus which, in particular, guarantees an effective and reliable washing of the internal parts of the items to be washed.

Another purpose of the present invention is to obtain a washing apparatus that allows to effectively subject to one or more wash cycles items of different shape and type, always guaranteeing effective cleaning both inside and outside said items.

Another purpose is to obtain an efficient device to support the items, for example, garments that can be used in hospitals.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, embodiments described here concern a washing apparatus. According to one embodiment, the washing apparatus comprises at least a washing chamber and at least a hydraulic circuit to deliver at least a treatment fluid inside the washing chamber.

According to one aspect of the invention, the washing apparatus comprises at least a support device able to be selectively housed in the washing chamber and comprising: at least a support unit, provided with a frame in the form of a cage and able to support items to be washed; and at least a delivery unit to deliver the treatment fluid configured to be hydraulically connected to the hydraulic circuit and provided with tubular elements configured to be positioned, at least partly, inside the frame, and therefore in the internal parts of the items to be washed.

The delivery unit of the treatment fluid can comprise first tubular elements with a vertical extension bigger than the extension of the frame.

The delivery unit of the treatment fluid can comprise second tubular elements with a vertical extension smaller than the extension of the frame.

According to another aspect, the delivery unit is hydraulically connectable to the hydraulic circuit by removable connection means.

In some embodiments, the delivery unit is hydraulically connectable to at least one tubular element on which at least one connection element is positioned, hydraulically associable with at least a mobile pipe of the hydraulic circuit.

According to other aspects, the tubular elements of the treatment fluid delivery unit are disposed so as to form a pronged structure.

Other embodiments concern a support device for items to be washed. According to a characteristic aspect, the device comprises: at least a support unit, provided with a frame in the form of a cage and able to support items to be washed, and at least a delivery unit to deliver a treatment fluid configured to be hydraulically connected to supply means of the treatment fluid and provided with tubular elements configured to be positioned at least partly inside the frame, and therefore in the internal parts of the items to be washed.

Other embodiments concern a method to wash items to be washed, wherein the items to be washed are positioned in a washing chamber and a treatment fluid is delivered inside the washing chamber by means of at least a hydraulic circuit. In accordance with one embodiment, the method provides that the items to be washed are supported using at least a support device that is housed inside the washing chamber and comprising: at least a support unit, provided with a frame in the form of a cage that supports the items to be washed; and at least a delivery unit of the treatment fluid that is hydraulically connected to the hydraulic circuit and provided with tubular elements positioned, at least partly, inside the frame, and therefore in the internal parts of the items to be washed, so that the items to be washed are washed both from the outside and from the inside by means of the treatment fluid.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the item of divisional applications.

It is understood that any aspect or characteristic that is discovered, during the patenting process, to be already known, shall not be claimed and shall be the item of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a front view of the washing apparatus according to the invention;
- fig. 2 is a three-dimensional view of a support device for items or clothing insertable in the washing apparatus;
- fig. 3 is a three-dimensional view of one of the support units for items or clothing provided in the support device in fig. 2.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

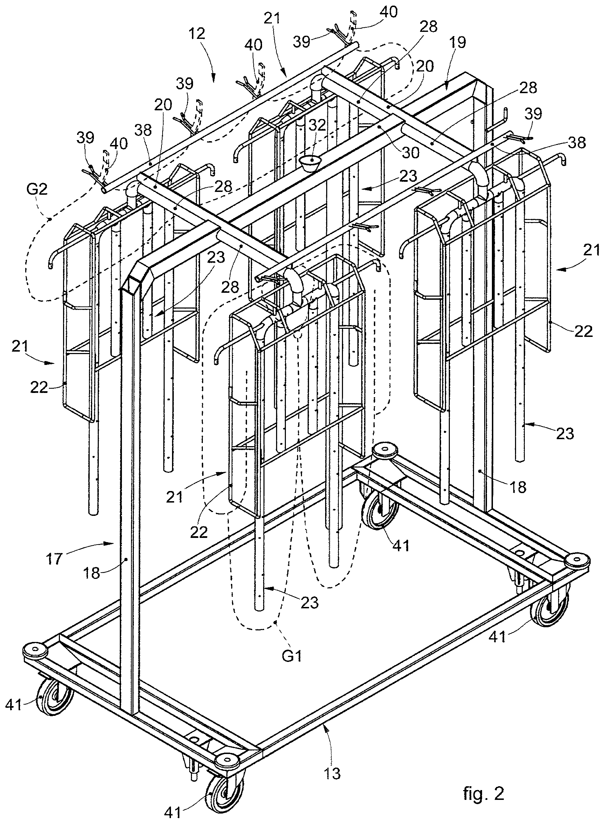

A washing apparatus 10 according to the present invention comprises a washing chamber 11 in which the items to be washed, such as items G1 and G2, are introduced, see the lines of dashes in fig. 2.

The items G1 and G2 to be washed are in this example garments used in healthcare, hospitals, or suchlike.

For example, one might hypothesize that item G1 is a gown used for example in a hospital's radiology department.

For example, one might hypothesize that item G2 is a skirt or suchlike, also used in a hospital's radiology department.

Items G1 and/or G2, however, could also be of another type and used in different health, hospital, or similar areas.

Items G1 and G2 are positioned on a support device 12.

Preferably, the support device 12 comprises a trolley base 13, provided with castors or wheels 41, so that it can be easily inserted into and removed from the washing chamber 11.

The washing chamber 11 can be the oblong tunnel type and is delimited by two lateral walls 14, a bottom wall, a ceiling 15, and a floor 16.

The washing chamber 11 is also provided with access apertures, which can be closed by means of doors of a known type, for example of the sliding type.

A first aperture, disposed on the front side of the washing chamber 11, will be used for the entry of the support device 12 into said washing chamber 11 from the "dirty" side, that is, prior to washing.

A second aperture, disposed on the rear side, will be used for the exit of the washing device 12 from the washing chamber 11 on the clean side.

The washing chamber 11 can also be predisposed and sized to accommodate several support members 12, located in sequence.

If, for example, it accommodates two support devices 12, the support devices will be aligned with the entry and exit apertures of the washing chamber 11.

The washing apparatus 10 also comprises a hydraulic circuit 33 able to distribute the washing liquid inside the washing chamber 11. The hydraulic circuit 33 is connected to a series of washing lances of a known type, disposed inside the chamber, laterally, on the bottom and at the top.

By means of the hydraulic circuit 33 and the lances it is possible to deliver a treatment fluid in particular on the external surfaces of the garments to be washed.

The hydraulic circuit 33 is also provided with at least one fixed pipe 34 and at least one mobile pipe 35, the functions of which will be explained later.

On the base 13 of the support device 12 shown in the drawings, a support frame 17 is positioned, provided with a pair of uprights 18 and a longitudinal element 19 to join the uprights 18.

The frame 17 is provided in this example with a pair of cross-pieces 20.

In correspondence with each end of each cross-piece 20, a support unit 21 is attached, able to accommodate a G1 item to be washed and/or part of an item G2 to be washed.

Each support unit 21 comprises a positioning frame 22 of the item G1 or G2, able to guarantee that the item G1 or G2 to be washed is correctly disposed.

The frame 22, as can be seen from the drawings, can be in the form of a cage or suchlike, so that the items to be treated are correctly positioned, spread out or lying on it.

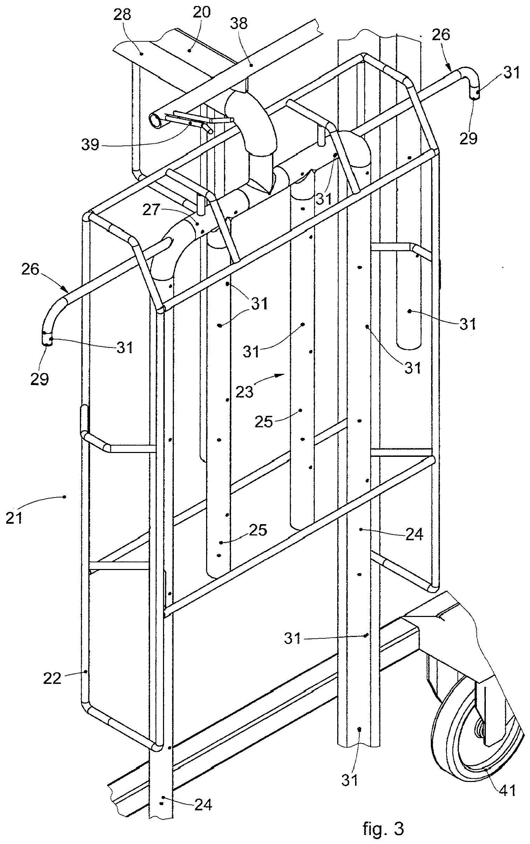

The support unit 21 also comprises a delivery unit 23 to deliver a treatment fluid inside the items G1 or G2 to be washed, see also fig. 3.

The treatment fluid delivery unit 23 comprises for example a series of tubular elements 24-27 in reciprocal hydraulic communication and able to allow a correct, complete and efficient washing cycle of items G1 and G2.

Advantageously, the tubular elements 24-27 of the treatment fluid delivery unit 23 are integrated in the aforementioned frame 22.

First tubular elements 24, disposed substantially in a vertical direction and having a suitable extension, are able to completely wash the entire length of a given item or garment, for example an item G1.

The first tubular elements 24 can have a bigger vertical extension than the extension of the support unit 21, and therefore of the relative frame 22.

The extension of the tubular elements 24 can be provided, for example, in order to allow a correct internal treatment of garments of a certain length, such as for example the garment G1 shown, which is substantially provided with a trouser where the tubular elements 24 are inserted, see fig. 2.

Second tubular elements 25, disposed substantially in a vertical direction and for example in a more internal position with respect to the first tubular elements 24, are able to completely and effectively wash item G1 internally and in correspondence with the bust.

For example, it can be provided that the second tubular elements 25 have a smaller extension than the first tubular elements 24.

Third tubular elements 26, disposed substantially in a horizontal direction and possibly provided, at the free ends, with substantially vertical appendices 29, are provided to actuate an effective internal wash of the shoulders and sleeves of item G1.

The tubular elements 24, 25 and 26 are connected to another tubular connection element 27.

The tubular connection element 27 of the treatment fluid delivery unit 23 is in hydraulic connection with at least a substantially transverse pipe 28.

The pipe 28 connected to each of the delivery units 23 is connected to the longitudinal element 19 which comprises a tubular member 30, at least in the segment comprised between the two pipes 28, so that it can be hydraulically connected with each of the pipes 28.

The tubular elements 24-27 of the delivery unit 23 and the appendixes 29 are provided with nozzles or holes 31, which are variously distributed, to deliver the treatment fluid.

The tubular elements 24-27 of the delivery unit 23, in the example shown in the drawings, substantially form a pronged structure.

In the example shown, the tubular elements 24-27 are directed substantially in a vertical direction and in a horizontal direction. Optionally or alternatively to what is shown, it would be possible to provide tubular elements directed diagonally at any angle whatsoever, useful to allow an effective delivery of the treatment fluid.

Therefore, in the delivery unit 23 it is possible to provide tubular elements to deliver treatment fluid in any number and with any orientation.

The tubular element 30 of the longitudinal element 19 comprises at least one connection element 32, for example in the form of a funnel or cone, which opens inside the tubular element 30.

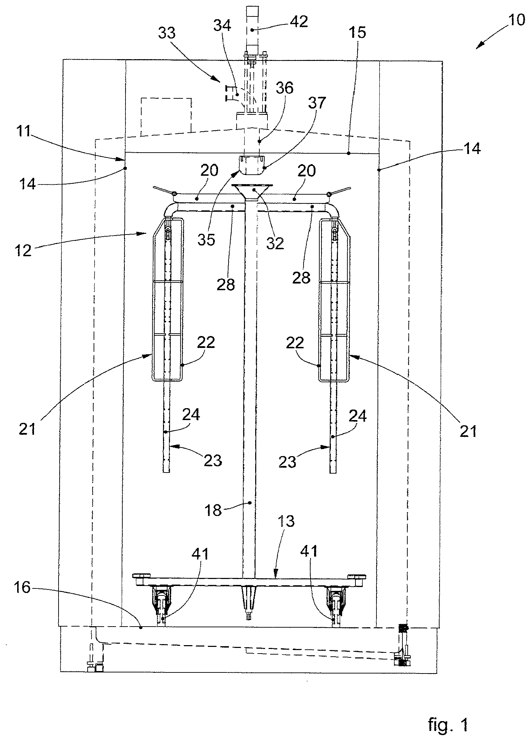

The connection element 32 is able to be connected, see fig. 1, with the mobile pipe 35 of the hydraulic circuit 33.

It can be provided that the mobile pipe 35 is configured to be inserted telescopically in the fixed pipe 34 of the hydraulic circuit 33.

If there are several support devices 12, it is provided that each support device 12 has its own connection element 32, therefore if several support devices 12 located in sequence are introduced into the washing chamber 11, it is necessary to provide several mobile pipes 35 that can be associated on one side to several fixed pipes 34 and on the other side to the corresponding connection elements 32.

Continuing in the example shown, relating to a support device 12, the mobile pipe is preferably positioned near the ceiling 15 of the washing chamber 11, in a position coordinated for the insertion into the respective connection element 32 of the support device 12.

The mobile pipe 35 is made selectively mobile between a first position in which it is hydraulically connected to the connection element 32 and a second position in which it is distanced and separated from the connection element 32 as shown in fig. 1.

When the hydraulic circuit 33 is connected to the element connection 32, therefore when the mobile pipe 35 is in the connection element 32, the apparatus 10 can deliver a suitable treatment fluid to each of the delivery units 23 of the support device 12.

In order to be moved, the mobile pipe 35 of the hydraulic circuit 33 is able to be connected to a piston 36 of an actuator 42, so as to be moved, in a substantially vertical manner, toward the inside of the washing chamber 11, and vice versa.

The mobile pipe 35 is preferably provided with a pipe union 37 which can be inserted into the connection element 32.

The pipe union 37 can be made, for example, of POM, Teflon or other plastic material with similar properties, able to be inserted into the connection element 32 for the hydraulic connection.

The pipe union 37 has a substantially cylindrical shape with a connection profile between the lateral surface and the rounded lower surface, so as to allow an effective coupling with the connection element 32.

Alternatively, the pipe union 37 can be conical, truncated cone or other suitable shape.

The support device 12 also comprises other support bars 38.

The support bars 38 are disposed above the support units 21 and comprise a series of attachment elements 39, useful for providing a further positioning mode for the garments to be washed.

The garment or item G2, for example, could be provided with buckles 40 able to be suitably inserted into the attachment elements 39 so as to keep it in a fully open position where it is washable effectively.

The apparatus 10 is also provided with a control and adjustment unit for the washing cycle and in general the feed and delivery of the treatment fluid to and from the hydraulic circuit 33.

The apparatus 10 as described heretofore functions as follows.

At the beginning of a new washing cycle, at least one support device 12, on which the garments to be subjected to washing are disposed, including for example, items G1 and G2, is thrust inside the washing chamber 11 from the "dirty" side of the washing chamber 11, until the mobile pipe 35 of the hydraulic circuit 33 is located in correspondence with the connection element 32.

The correct positioning can be guaranteed by any positioning device whatsoever, for example a pin which can be associated to one of the wheels 41 of the base 13, or other.

When the correct positioning of the support device 12 has been completed, an operator provides to start the wash cycle of the apparatus 10. The control unit activates the command to close the access door on the "dirty" side and commands the movement of the mobile pipe 35, thrusting the pipe union 37 inside the connection element 32.

During the washing cycle, the actuator 42 exerts a constant vertical thrust able to maintain the insertion and hydraulic coupling of the pipe union 37 in the connection element 32.

Once the washing cycle is started, the treatment fluid relating to each washing step is sent from the hydraulic circuit 33 to the fixed pipe 34 and then to the mobile pipe 35.

The treatment fluid is then introduced into the tubular element 30 and flows from here, through the pipes 28, to each of the delivery units 23, able to deliver, advantageously and by means of the various tubular elements 24-27, the treatment fluid in particular into the internal parts of the items, in particular the garments, to be washed.

The tubular element 30, the pipes 28 and the delivery units 23 of each support unit 21, substantially form a further hydraulic circuit able to receive the treatment fluid from the main hydraulic circuit 33 and to allow an effective internal washing of the items.

The connection element 32, selectively associable with the mobile pipe 35 of the hydraulic circuit 33, represents one of the many examples of removable hydraulic coupling means of the hydraulic circuit 33 with each of the delivery units 23.

By treatment fluid we mean, for example, a prewash, washing, rinsing or thermodisinfection fluid or liquid, or a drying fluid, depending on the steps of the washing cycle performed.

Naturally, the control unit can command the delivery of the treatment fluid also from the lances provided in the walls of the washing chamber 11, to wash the outside of the items.

At the end of the washing cycle, in particular after washing the internal part of the items, the control unit commands the lifting of the mobile pipe 35, and hence the separation of the pipe union 37 from the connection element 32.

When the washing cycle is completed, the control unit can command the opening of the door, for example a sliding door, located in correspondence with the aperture on the "clean" side: the support device 12 with the clean items can then be extracted from the washing chamber 11.

In the case of two or more support devices 12 located in sequence and with corresponding items, in particular garments to be washed, it is possible to provide that once a first support device has been unloaded, a second support device is automatically and correctly positioned and the washing cycle of the corresponding items is started, as described above.

The movement of the support device 12, or support devices, can be manual or automated, as shown in the Italian patents in the name of the Applicant, IT-B-1359341 and IT-B-1370325, incorporated herein in their entirety as a reference.

Furthermore, the washing chamber 11 can have two apertures and two corresponding doors, in a "first-in first-out" cycle, or a single aperture with a single door through which the support devices 12 enter and exit, in a "last-in first-out" cycle.

Another variant provides to feed two support devices 12 or more in parallel.

The support device 12 could naturally also accommodate a different number of support units 21, compared with what is shown here.

The support device 12 could for example provide one support unit 21 on every side or more than two support units 21 on every side. For every pair of support units 21 disposed on opposite sides with respect to the longitudinal element 19, therefore, a support cross-piece 20 will be provided.

It would also be possible to provide a support device 12 comprising one or more support units on one side only of the support frame 17, or a support device 12 having a different number of support units 21 on every side.

It is clear that modifications and/or additions of parts may be made to the washing apparatus 10 as described heretofore, without departing from the field and scope of the present invention.

It is also clear that, although the present invention has been described with reference to some specific examples, a person of skill in the art shall certainly be able to achieve many other equivalent forms of washing apparatus, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Washing apparatus comprising at least a washing chamber (11) and at least a hydraulic circuit (33) to deliver at least a treatment fluid inside the washing chamber (11), said washing apparatus being **characterized in that** it comprises at least a support device (12) able to be housed in said washing chamber (11) and comprising: at least a support unit (21), provided with a frame (22) in the form of a cage and able to support items (G1, G2) to be washed; and at least a delivery unit (23) to deliver said treatment fluid configured to be hydraulically connected to said hydraulic circuit (33) and provided with tubular elements (24-27) configured to be positioned, at least partly, inside said frame (22), and therefore in the internal parts of the items (G1, G2) to be washed.

2. Washing apparatus as in claim 1, **characterized in that** said delivery unit (23) of the treatment fluid comprises first tubular elements (24) with a vertical extension bigger than the extension of said frame (22).

3. Washing apparatus as in claim 1 or 2, **characterized in that** said delivery unit (23) of the treatment fluid comprises second tubular elements (25) with a vertical extension smaller than the extension of said frame (22).

4. Washing apparatus as in any claim hereinbefore, **characterized in that** said delivery unit (23) is hydraulically connectable to said hydraulic circuit (33) by removable connection means (32, 35).

5. Washing apparatus as in claim 4, **characterized in that** said delivery unit (23) is hydraulically connectable to at least one tubular element (30) on which at least one connection element (32) is positioned, hydraulically associable with at least a mobile pipe (35) of the hydraulic circuit (33).

6. Washing apparatus as in any claim hereinbefore, **characterized in that** said support device (12) comprises a trolley base (13).

7. Washing apparatus as in any claim hereinbefore, **characterized in that** said tubular elements (24-27) of the delivery unit (23) are provided with holes or nozzles (31) to deliver the treatment fluid.

8. Washing apparatus as in any claim hereinbefore, **characterized in that** said tubular elements (24-27) of the treatment fluid delivery unit (23) substantially form a pronged structure.

9. Washing apparatus as in any claim hereinbefore, **characterized in that** said tubular elements (24-27) of the delivery unit (23) are made in a plurality of different lengths and are disposed in a plurality of different directions, so that the treatment fluid can reach substantially every internal zone of the item to be washed.

10. Washing apparatus as in any claim hereinbefore, **characterized in that** said support device (12) comprises one or more support bars (38), configured and positioned so as to allow to efficiently lay the items (G1, G2) to be washed on one or more support units (21) and on one or more treatment fluid delivery units (23).

11. Support device for items to be washed, **characterized in that** said support device comprises: at least a support unit (21), provided with a frame (22) in the form of a cage and able to support items (G1, G2) to be washed and at least a delivery unit (23) to deliver a treatment fluid configured to be hydraulically connected to supply means of said treatment fluid and provided with tubular elements (24-27) configured to be positioned at least partly inside said frame (22), and therefore in the internal parts of the items (G1, G2) to be washed.

12. Method to wash items to be washed, wherein the items to be washed (G1, G2) are positioned in a washing chamber (11) and a treatment fluid is delivered inside the washing chamber (11) by means of at least a hydraulic circuit (33), said method being **characterized in that** the items to be washed (G1, G2) are supported using at least a support device (12) that is housed inside said washing chamber (11) and comprising: at least a support unit (21), provided with a frame (22) in the form of a cage that supports the items (G1, G2) to be washed; and at least a delivery unit (23) to deliver said treatment fluid that is hydraulically connected to said hydraulic circuit (33) and provided with tubular elements (24-27) positioned, at least partly, inside said frame (22), and therefore in the internal parts of the items (G1, G2) to be washed, so that the items to be washed (G1, G2) are washed both from the outside and also from the inside by means of said treatment fluid.
